# EUROPEAN PATENT SPECIFICATION

(11) **EP 0 602 290 B1**
(45) Date of publication and mention of the grant of the patent: **25.08.1999**
(21) Application number: 92311109.0
(22) Date of filing: 04.12.1992
(51) Int. Cl.: A61K 47/48

(54) **Antibody-conjugated Hepatitis B surface antigen and use thereof**
Antikörper-konjugiertes-Hepatitis B-Oberflächenantigen und dessen Verwendung
Antigène de surface du virus de l'hépatite B conjugué à des anticorps et utilisation d'un tel conjugué

(43) Date of publication of application: 22.06.1994
(73) Proprietor: ConjuChem, Inc., Montreal, Québec (CA)
(72) Inventor: Pouletty, Philippe, Atherton, CA 94027 (US); Pouletty, Christine, Atherton, CA 94027 (US)
(74) Representative: Harrison, David Christopher

(56) References cited:
- EP-A- 0 066 296
- EP-A- 0 425 235
- EP-A- 0 510 949
- WO-A-86/01409
- WO-A-89/08261
- WO-A-90/11783
- WO-A-90/12585
- WO-A-92/12729
- DE-A- 4 014 540
- STN FILE SUPPLIER & FILE MEDLINE AN=88:28853 (KARLSR.) & CLIN. EXP. IMMUNOL. vol. 72, no. 1, April 1988, pages 164 - 167; S.RATH ET AL.: 'IGC SUBCLASS COMPOSITION OF Ab TO HBSAG IN CIRCULATING IMMUNE COMPLEXES FROM PATIENTS WITH HEPATITIS B VIRUS INFECTIONS.'
- STN FILE SUPPLIER & FILE BIOSIS AN=86:142723 (KARLSR.)
- IMMUNOL. CLIN. SPER. vol. 4, no. 3, 1985, pages 177 - 188 (A. CRAXI ET AL. 'IN-VITRO CYTOTOXICITY TO HEPATITIS B SURFACE ANTIGEN-COATED AUTOLOGOUS TARGET CELLS IN HEPATITIS B VIRUS INFECTION.'
- STN FILE SUPPLIER (KARLSR.) & CHEMICAL ABSTRACTS, vol. 115, no. 5, Columbus, Ohio, US; abstract no. 47345z, & J. IMMUNOL. METHODS vol. 138, no. 1, 1991, pages 111 - 119; K. M. WILSON: 'RAPID WHOLE BLOOD ASSAY FOR HIV-1 SEROPOSITIVITY USING AN FAB-PEPTIDE CONJUGATE'

## Description

### Technical Field

The field of this invention is drug delivery systems.

### Background

The administration of drugs provides many challenges. Depending upon the nature of the drug, the drug may be directed preferentially to different sites in the body, may be cleared more or less rapidly, may bind to a variety of proteins, may be subject to degradation and catabolism, renal clearance, or the like. While in some cases, there is an interest in having rapid clearance of the drug, in most situations, it is desired to have a therapeutic blood concentration over long periods of time.

In order to maintain the blood level at or above the therapeutic concentration, drugs are frequently administered at dosages substantially above the therapeutic dosage or administered incrementally at levels below the therapeutic dosage at repetitive time intervals, or administered repeatedly for long periods of time to maintain the therapeutic blood concentration until the therapeutic dosage is achieved. Neither of these situations is particularly attractive, since in most cases the drug will have side effects, so that the side effects are potentiated at the elevated drug dosages. Furthermore, where the drug is administered sequentially, until a therapeutic dosage is achieved, the patient is not benefiting from the presence of the drug, so that there may be an extended time period between the time when drug administration begins and the time when the patient is obtaining some benefit from the drug. Also, frequent administration of a drug, especially by parenteral administration, implies a high level of health care, higher cost, and lower comfort for the patient.

WO90/11783 in the name of Centocor, Inc. discloses platelet specific immunoconjugates. These immunoconjugates comprise a platelet-specific antibody coupled to a thrombolytic agent for use in anti-thrombotic therapy.

CA 115:47345Z (J.Immunol.Methods vol. 138 No.1) teaches a rapid whole blood test developed for circulating antibodies to human immunodeficiency virus type 1 (HIV-1), based on agglutination of autologous red blood cells. It discloses an anti-red blood cell Fab conjugated to a synthetic peptide corresponding to the immunodominant epitope of the HIV-1 viral coat protein.

DE-A-4014540 in the name of Tschaikowsky K teaches the use of immune conjugates for prophylaxis and treatment of organ damage - caused by inflammation containing a monoclonal antibody, cross linker and enzyme inhibitor of protein kinase C or calcium antagonist.

EP 425235 in the name of Immunogen Inc. relates to a cytoxic agent comprising one or more maytansinoids linked to a cell binding agent. The cell binding agent may be a monoclonal antibody.

EP510949 discloses conjugates for inactivating a target cell wherein the conjugate comprises a moiety specific for a surface membrane receptor.

WO90/12585 in the name of Oncogen Limited Partnership discloses Oncostatin M covalently conjugated to an antibody that defines a cellular antigen for increasing plasmin activity.

A wide variety of techniques have been suggested to improve modes of drug administration. One technique involves therapeutic dosage monitoring, where a large bolus of the drug is initially given to bring the patient rapidly to the therapeutic dosage level and the level of drug in the bloodstream is monitored, so as to ensure that the dosage is maintained at an adequate effective dosage. Quite clearly, this is expensive in requiring repetitive sampling and monitoring of drug levels in the host. This is only feasible with those drugs, where chronic administration is provided and the drug is monitored at relatively extended intervals. Other techniques have involved slow release capsules or particles, where the capsules or particles continuously release drug. The particles create numerous problems, being ingested by macrophages, being subject to rapid clearance, and being difficult to regulate.

There is, therefore, substantial interest in being able to provide for improved methods of administering drugs, where the drug will have an extended half-life in the patient, may be relatively uniformly distributed throughout the vascular and/or lymphatic system or directed to a particular site and allow for ease of administration.

### SUMMARY OF THE INVENTION

Physiologically active compounds are administered conjugated to receptors which bind to surface membrane proteins of erythrocytes, platelets or endothelial cells, where in the case of erythrocytes and platelets, the conjugates may be administered bound to the cells. Specifically, the compound, usually a drug, is conjugated to a monoclonal antibody or fragment, where the antibody fragment thereof is specific for a target epitope of the indicated calls. The conjugates are conveniently administered parenterally.

### DESCRIPTION OF THE SPECIFIC EMBODIMENTS

Methods and compositions are provided for the administration in vivo to mammalian hosts, particularly primate hosts, of a wide variety of physiologically active compounds, where prolonged availability of the compound to achieved. The method employs a conjugate of the compound, usually a drug, with a receptor having a high affinity for erythrocytes, platelets or endothelial cells, where the site of conjugation is at other than the binding site so as to retain binding affinity.

The receptor will, for the most part, be a monoclonal antibody or fragment thereof, which is specific for an epitope substantially specific for an erythrocyte, platelet or endothelial cell.

The antibody or fragment thereof may be any one of the subtypes or isotypes, only being one that does not participate in complement effected lysis. Therefore, the whole antibody may be IgA, IgD, IgG₁ or IgG₄ or corresponding isotypes of other species, while fragments may be from any isotype. Normally, the antibody will be one which is allogenic, although in some instances, xenogenic antibodies may be employed. Particularly, xenogenic antibodies may be employed in those situations where the host is immunocompromised, or the antibody or antibody fragment is non-antigenic, despite its being xenogeneic, or one wishes to enhance the antigenicity of the physiologically active compound.

The whole antibody need not be used, fragments being frequently useful, such an Fᵥ, Fab, F(ab')₂, the heavy chain, a single-chain antigen binding protein, or the like. The fragment which is used, should retain at least a substantial portion, better than about fifty percent (50%) of the original affinity of the antibody.

The binding site of the antibody should be relatively specific for the target cell. That is, better than about fifty percent (50%), preferably more than about seventy-five percent (75%) of the antibody introduced to the host, which binds to antigens will bind to the target cell. In this way, one can assure that a major proportion of the conjugate will be present over extended periods of time and will not be endocytosed, cleared from the blood stream, or the like.

Monoclonal antibodies may be obtained in accordance with conventional ways. For example, for monoclonal antibodies specific for erythrocytes, lymphocytes may be collected from an individual with a positive direct Coombs test, particularly of the IgG type, (without complement) without a showing of hemolysis. The lymphocytes may be immortalized by any convenient means, e.g. Epstein-Barr virus transformation, followed by screening for antibodies having the desired specificity and affinity, an well as the desired isotype. The antibodies may then be modified in a variety of ways, by enzymatic cleavage to provide for the fragments, using papain, chymotrypsin, pepsin, reduction with cleavage of intramolecular disulfide linkages or the like.

The monoclonal antibodies may be from any source, such as primate, particularly human, murine, lagomorpha, canine, ovine, porcine, equine, or the like.

Alternatively, polyclonal antibodies rained against a fragment of the target antigen, such as a synthetic peptide, may be used.

Antibodies which bind to erythrocytes may be further characterized by binding to red cells, particularly O Rh negative red cells, and to panels of red cells of known phenotypes. Those cells which react with all the cells of the panel and, furthermore, show negative results with the direct Coombs test using anti-complement globulin are selected. Ref: Stratton, F.; Rawlinson, Vi; Merry, A.H.; Thomson, E.E.; Clin. Lab. Haematol. 1983, 5:17-21. In addition, the antibodies are screened as to cross-reactivity with other cell types, such a lymphocytes, myelomonocytes, and the like. Similarly, for antibodies specific for platelets or endothelial cells, the antibodies are selected to be specific for such cells and not for other cells which may be encountered in the host. For the most part, with erythrocytes the number of conjugates will be below the level that causes hemolysis, usually fewer than about 5 x 10³/cell.

In some situations activation of the target cell will result in up-regulation of a surface membrane protein. For endothelial Cells, addressins, e.g. ELAM-1, or integrins, e.g. VLA-4, may be up-regulated in the case of inflammation. In this way, the drug may be localized as the site of inflammation, providing for a localized concentration at the target region.

The cells which are selected have a number of advantages. These cells are present in high number in the vascular system. Platelets are present in from about 1-4x10⁵/µl, while erythrocytes are present in about 4-6x10⁶/µl. The cells have a long half-life and by appropriate choice of epitope, endocytosis is avoided. The red cells and platelets lack a nucleus and cell division capability. All of the cells have a wide distribution in capillaries and tissue and express specific epitopes associated with their specific differentiation. Furthermore, for the erythrocytes and platelets, those cells may be readily collected, combined with the conjugate, and administered to the host. The cells may be autologous or allogenic.

If desired, the antibodies can be prepared or modified in a variety of ways. Chimeric antibodies may be prepared, where the constant region may be modified as to isotype or species. For example, murine monoclonal antibodies may be prepared, the genes encoding the heavy and light chains isolated, and the constant regions of the heavy and light chains substituted with the appropriate constant regions of human constant regions to provide for a chimeric antibody which lacks the antigenicity of the murine constant region Alternatively, variable regions obtained from a host may be cloned and mutated and then screened to identify specific binding affinities. A further alternative is to exchange not only the constant region of the heavy and light chain, but also exchange the framework regions of the variable domains, so as to further reduce the antigenicity of the antibody. Numerous techniques are described in the literature, for example, "The synthesis and in vivo assembly of functional antibodies in yeast," Wood, CR., Boss, M.A., Kenten, J.H., Calvert, J.E., Roberts, N.A., Emtage, J.S., Nature, Apr 4-10, 1985, 314(6010):446-9; "Construction of chimaeric processed immunoglobulin genes containing mouse variable and human constant region sequences," Takeda, S., Naito, T., Hama, K., Noma, T., Honjo, T., Nature, Apr 4-10, 1985, 314(6010):452-4; "A recombinant immunotoxin consisting of two antibody variable domains fused to Pseudomonas exotoxin," Chaudhary, V.K., Queen, C., Junghns, R.P., Waldmann, T.A., FitzGerald, D.J., Pastan, I, Laboratory of Molecular Biology, DCBD, National Cancer institute, Bethesda, Maryland 20892, Nature, Jun 1, 1989, 339(6223):394-7; "Binding activities of a repertoire of single immunoglobulin variable domains secreted from *Escherichia coli* [see comments]," Ward, E.S., Gussow, D., Griffiths, A.D., Jones, P.T., Winter, G., MRC Laboratory or Molecular Biology, Cambridge, UK, Nature (ENGLAND), Oct 12, 1989, 341(6242):544-6, ISSN 0028-0836, Comment in Nature, 1989, Oct 12, 341(6242):484-5.

The physiologically active compounds which are conjugated to the receptor will vary widely depending upon the nature and the purpose of the physiologically active compound. In the case of vaccines, the physiologically active compound may be an antigen or immunologically active fragment thereof associated with a pathogen, e.g., virus, bacteria, protozoa, fungus; an allergen, or other parasite or debilitating antigen or an histocompatibility antigen to enhance antigen presentation. Preferably, the physiologically active compound is Hepititis B surface antigen. By providing for an antigen which binds to the cells, the lifetime of the antigen in the blood stream may be greatly extended, so as to continuously stimulate the immune system over an extended period of time.

The nature of the linkage between the physiologically active compound and the receptor will vary widely depending upon the nature of the physiologically active compound, whether one wishes to retain it bound to the receptor or allow for its release into solution, and the rate at which one wishes to provide for the release. A wide variety of linkages will be unstable under physiological conditions, where the instability may be as a result of enzymatic or non-enzymatic reactions. For example, ester linkages can be provided which are relatively labile and will be cleaved over time in the blood stream. By providing for varying degrees of steric hindrance, different degrees of lability can be achieved. Alternatively, sequences can be provided, which are recognized by a wide variety of proteases. For example, a series of arginine-lysine dimeric units will be sensitive to trypsin. Other protease labile linkages may be employed, where the protease may be found in the bloodstream. Alternatively, one may employ disulfide linkages, which will be subject to reductive cleavage. Other functionalities which may find use include oligosaccharides, thiol eaters, nucleic acids, or the like. (By "stable" is intended that the linkage will not be preferentially cleaved as compared to other bonds in the molecule, "unstable" intends preferential cleavage.)

There are extensive reports in the literature of procedures for covalently joining a wide variety of compounds to prepare conjugates with different functional groups. Various synthetic schemes may be envisioned, depending upon the nature of the physiologically active compound. Thus, where sulfhydryl groups are present, these may be readily activated to provide for linkage to a sulfhydryl group to provide a disulfide. Carboxyl groups may be readily activated with a wide variety of hydroxyl compounds or carbodiimide, where the ester or anhydride is allowed to react with hydroxyl or amino groups to provide esters or amides. Other linkages may also find application, such as imines, hydrazines, etc.

The antibody fragment and the active compound may be genetically engineered as a single construct, cloned and expressed using a variety of vectors and host cells. Alternatively, both can be chemically synthesized.

Depending on the nature of the physiologically active compound, on the average, less than one, or one or more molecules of the physiologically active compound may be conjugated to the receptor. In some instances, it may be desirable to modify the receptor so as to provide for a target functionality which may be used to link the physiologically active compound. For example, one may introduce a maleimide group, where a thiol may bind to the maleimide to provide for a thioether. By employing a disulfide, one will then provide for a disulfide linkage.

The subject compositions may be administered in a wide variety of ways. If desired, the conjugate may be first bound to the cells in appropriate proportion followed by administration of the conjugate-bound cells to the host. Alternatively, the conjugate may be administered to the host for binding to the target cells. The amount of the conjugate which is administered will vary widely, depending upon the nature of the conjugate, the purpose for the conjugate, the therapeutic dosage, the physiological activity of the compound when present as a conjugate, and when bound to a cell, and the like. Therefore, the amount of conjugate administered to a host may vary from 1 pg to 10 mg/kg of host.

The subject compositions will, for the most part, be administered parenterally, such as intravascularly, intraarterially, subcutaneously, or the like. For the most part, physiologically active carriers will be employed, such as water, saline, phosphate buffered saline, aqueous ethanol, plasma, proteinaceous solutions, glucose solutions, or the like. The concentration of the conjugate will vary widely, generally ranging from about 1 pg/ml to 10 mg/ml. Other additives which may be included include buffers, where the media are generally buffered at a pH in the range of about 5 to 10, where the buffer will generally range in concentration from about 50 to 250 mM, salt, where the concentration of salt will generally range from about 5 to 500 mM, physiologically acceptable stabilizers, and the like.

For vaccines, the physiologically active compound may be any protein of the pathogen which results in neutralizing antibodies, such as envelope proteins, capsid proteins, surface membrane proteins, cell wall proteins and saccharides, and the like. Preferably, the physiologically active compound is Hepititis B surface antigen.

The following examples are offered by way of illustration and the way of implementation.

### EXPERIMENTAL

Human monoclonal antibodies ware derived from an EBV transformation of lymphocytes collected from an individual with a positive direct Coombs test. This individual had a positive direct Coombs test of the IgG type (without complement) and showed no sign of hemolysis. Anti-rod cell antibodies wore selected by liquid phase ELISA and indirect Coombs test using O Rh negative red cells, and further characterized using panels of red cells of known phenotypes. Only monoclonal antibodies reacting with all cells of the panels and showing negative results with the indirect Coombs test using anti-complement globulin were selected. Cell cross-reactivity was further tested by a cytofluorometric method against various cells types (T and B cells, platelets, monocytes, polynuclear cells) to check for non-cross reactivity. Monoclonal antibody isotype was tested by ELISA using monospecific antibodies. Briefly, monoclonal antibody specimens were incubated in microtiter plate wells coated respectively with anti-IgG₁, -IgG₂, -IgG₃, -IgG₄, -IgM, -IgA goat specific antibodies, after washing and incubation with an anti-human immunoglobulin goat antibody peroxidase conjugate and washing. Color development following addition of o-phenylene diamine was measured spectrophotometroically at 495 mm.

Several anti-red blood cell monoclonal antibodies (IgG₄) were selected: CHP1, CHP2 and CHP3. Fab and F(ab')₂ fragments were made.

The subject antibody (CHP2) is conjugated to recombinant purified HBsAg using p-maleimidobenzoic acid. The acid is activated with carbodiimide and combined with HBsAg in stoichiometric ratio. The product is then reacted directly with Fab to provide the conjugate, which is then purified on HPLC. The subject conjugate is then administered to a mammalian host to induce an immune response to HBsAg.

A 5 mg dose of purified, sterile, conjugate was administered intravenously to one chimpanzee (C23). Control chimpanzees were as follows: C24 was injected with an equivalent dose of antibody fragment conjugated to bovine serum albumin; C25 was vaccinated using the Hevac B (Trade Mark) vaccine (Pasteur Vaccin) according to the manufacturer's instructions; C26 received a 5 mg dose of irrelevant human IgG₄ antibody fragment conjugated to HBs using the same conjugation, purification and administration procedures as for the conjugate administered to C23. The following parameters were monitored before and weekly (for 16 weeks) after injection:

Hemolysis was monitored by:
- --: red blood cell count
- --: hemoglobin blood level
- --: hematocrit
- --: reticulocyte count (using a Coulter instrument)
- --: bilirubin serum level

Binding of antibodies, conjugate and complement to red blood cells was monitored by modification of the standard direct Coombs test as follows:
- --: Direct Coombs test, *Fab type* (using an anti-human IgG, Fab fragment specific anti-globulin)
- --: Direct Coombs test, *Complement type* (using an anti-human Complement specific anti-globulin)
- --: Direct Coombs test, *Fc type* (using an anti-human IgG, Fc fragment specific anti-globulin)
- --: Direct Coombs test, *HBs type* (using a goat anti-HBs specific anti-globulin)

Titration of Anti-HBs antibodies was performed by solid phase ELISA (Abbott Laboratories).

### Results

None of the chimpanzees showed any biological evidence of pathological hemolysis. C23 developed immediately after the conjugate injection a positive, Fab type, and HBs type, direct Coombs test, which became negative at week 8. C24 developed immediately after the conjugate injection a positive Fab type only (HBs type negative) direct Coombs test, which became negative at week 9. The direct Coombs test of the Fc and Complement type became weakly positive at week 7 (until week 9) for C23 and C24, probably reflecting the anti-Human Fab and anti-HBs (C23) or the anti-Human Fab and anti-BSA (C24) antibody response. The direct Coombs tent remained negative for C25 and C26.

Anti-HBs antibodies were detected in C23 at week 3 (1:200) with a peak end point titer of 1:3200 at week 17. Anti-HBs antibodies were detected in C25 at week 4 (1:100) and remained between 1:50 and 1:200 subsequently. No anti-HBs antibodies were detected in C24. Anti-HBs antibodies were detected in C25 at week 4, with a peak end-point titer of 1:6400 at week 15, 3 weeks after the third monthly injection.

It is evident from the above results, that the subject methodology provides for prolongation of the presence of a physiologically active compound in a mammalian host. In this manner, greatly enhanced immuno responses or prolonged treatment with a physiologically active compound may be achieved. In this way, the circulation dosage of a compound may be controlled, while maintaining a therapeutic dosage over an extended period of time.

## Claims

1. Use of a composition for the manufacture of a medicament for extending the *in vivo* half-life of Hepatitis B surface antigen in a patient to provide an enhanced immune response to Hepatitis B,
the composition comprising an antibody or fragment thereof specific for a cell specific epitope, said cell being an endothelial cell, erythrocyte or platelet, said antibody or fragment thereof covalently conjugated at other than the binding sites of said antibody or fragment thereof to Hepatitis B surface antigen.

2. Use according to claims 1 wherein said antibody is a monoclonal antibody.

3. Use according to claim 1 or claim 2 wherein the said the cell is an endothelial cell.

4. Use according to claim 1 or claim 2 wherein the cell is an erythrocyte.

5. Use according to claim 1 or claim 2 wherein the cell is a platelet.

6. Use according to any one of claims 1 to 5 wherein the said antibody or fragment thereof is conjugated through a linkage stable under physiological conditions.

7. A compound comprising an monoclonal antibody or fragment thereof covalently conjugated at other than the binding site of said antibody or fragment to Hepatitis B surface antigen, wherein said antibody or fragment thereof is specific for a cell specific epitope, said cell being an endothelial cell, erythrocyte or platelet.

8. A compound according to claim 7 wherein the cell is an endothelial cell.

9. A compound according to claim 7 wherein the cell is an erythrocyte.

10. A compound according to claim 7 wherein the cell is a platelet.

11. A compound according to any of claims 7 to 11 being a drug.

12. A compound according to claim 11 wherein the drug is a naturally occurring compound or physiologically active fragment thereof.

13. A compound according to any of claims 7 to 12 wherein the antibody is IgG.

## Patentansprüche

1. Verwendung einer Zusammensetzung zur Herstellung eines Medikaments zur Verlängerung der in vivo-Halbwertszeit von Hepatitis B-Oberflächenantigen in einem Patienten, um eine verstärkte Immunreaktion auf Hepatitis B zu bewirken,
wobei die Zusammensetzung einen Antikörper oder ein Fragment davon enthält, der bzw. das für ein zellspezifisches Epitop spezifisch ist, wobei die Zelle eine Endothelzelle, ein Erythrozyt oder ein Blutplättchen ist, wobei der Antikörper oder dessen Fragment kovalent an anderen als den Bindestellen des Antikörpers oder dessen Fragments an Hepatitis B-Oberflächenantigen gebunden ist.

2. Verwendung nach Anspruch 1, worin der Antikörper ein monoklonaler Antikörper ist.

3. Verwendung nach Anspruch 1 oder 2, worin die Zelle eine Endothelzelle ist.

4. Verwendung nach Anspruch 1 oder 2, worin die Zelle ein Erythrozyt ist.

5. Verwendung nach Anspruch 1 oder 2, worin die Zelle ein Blutplättchen ist.

6. Verwendung nach einem der Ansprüche 1 bis 5, worin der Antikörper oder dessen Fragment über eine unter physiologischen Bedingungen stabile Bindung konjugiert ist.

7. Verbindung, umfassend einen monoklonalen Antikörper oder ein Fragment davon, der bzw. das kovalent an anderen als den Bindestellen des Antikörpers oder Fragments an Hepatitis B-Oberflächenantigen gebunden ist, wobei der Antikörper oder dessen Fragment für ein zellspezifisches Epitop spezifisch ist, wobei die Zelle eine Endothelzelle, ein Erythrozyt oder ein Blutplättchen ist.

8. Verbindung nach Anspruch 7, worin die Zelle eine Endothelzelle ist.

9. Verbindung nach Anspruch 7, worin die Zelle ein Erythrozyt ist.

10. Verbindung nach Anspruch 7, worin die Zelle ein Blutplättchen ist.

11. Verbindung nach nach einem der Ansprüche 7 bis 11, die ein Arzneimittel ist.

12. Verbindung nach Anspruch 11, worin das Arzneimittel eine natürlich vorkommende Verbindung oder ein pyhsiologisch aktives Fragment davon ist.

13. Verbindung nach einem der Ansprüche 7 bis 12, worin der Antikörper IgG ist.

## Revendications

1. Utilisation d'une composition pour la fabrication d'un médicament pour prolonger la demi-vie *in vivo* de l'antigène de surface de l'hépatite B chez un patient afin d'obtenir une réponse immunitaire améliorée à l'hépatite B,
la composition comprenant un anticorps ou l'un de ses fragments spécifique d'un épitope spécifique d'une cellule, ladite cellule étant une cellule endothéliale, un érychrocyte ou une plaquette, ledit anticorps ou son fragment étant conjugué par covalence, en un site différent des sites de liaison dudit anticorps ou de son fragment, à l'antigène de surface d'hépatite B.

2. Utilisation selon la revendication 1, dans laquelle le dit anticorps est un anticorps monoclonal

3. Utilisation selon la revendication 1 ou la revendication 2, dans laquelle ladite cellule est une cellule endothéliale

4. Utilisation selon la revendication 1 ou la revendication 2, dans laquelle la cellule est un érythrocyte.

5. Utilisation selon la revendication 1 ou la revendication 2, dans laquelle la cellule est une plaquette.

6. Utilisation selon l'une quelconque des revendications 1 à 5, dans laquelle ledit anticorps ou son fragment est conjugué par l'intermédiaire d'une liaison stable dans des conditions physiologiques.

7. Composé comprenant un anticorps monoclonal ou l'un de ses fragments, conjugué par covalence, en un site autre que le site de liaison dudit anticorps ou dudit fragment, à l'antigène de surface de l'hépatite B, dans lequel ledit anticorps ou son fragment est spécifique d'un épitope spécifique d'une cellule, ladite cellule étant une cellule endothéliale, un érythrooyte ou une plaquette.

8. Composé selon la revendication 7, dans lequel la cellule est une cellule endothéliale.

9. Composé selon la revendication 7, dans lequel la cellule est un érythrocyte.

10. Composé selon la revendication 7, dans lequel la cellule est une plaquette.

11. Composé selon l'une quelconque des revendications 7 à 11, qui est un médicament.

12. Composé selon la revendication 11, dans lequel le médicament est un composé naturel ou l'un de ses fragments physiologiquement actifs.

13. Composé selon l'une quelconque des revendications 7 à 12, dans lequel l'anticorps est une IgG.
